# DEMANDE DE BREVET EUROPEEN

(11) **EP 4 597 753 A1**
(43) Date de publication de la demande: **06.08.2025**
(21) Numéro de dépôt: 25154646.1
(22) Date de dépôt: 29.01.2025
(51) Int. Cl.: H01R 4/34, H02B 1/00, G01K 11/00, H02H 1/00, H01R 4/30, G01N 33/00, H01R 11/12

(54) **DISPOSITIF DE CONNEXION ÉLECTRIQUE, AINSI QU'APPAREIL ÉLECTRIQUE ET TABLEAU ÉLECTRIQUE ASSOCIÉS**

(30) Priorité: 30.01.2024 FR 2400884
(71) Demandeur: SCHNEIDER ELECTRIC INDUSTRIES SAS, 92500 Rueil-Malmaison (FR)
(72) Inventeur: AGNAOU, Abderrahmane, 38100 Grenoble (FR); LINARES, Louis, 38660 La Terrasse (FR)
(74) Mandataire: Lavoix

(57) **Abrégé**

La présente invention concerne un dispositif de connexion électrique entre un membre conducteur électrique (4A) et une plage de raccordement (6) d'un appareil électrique (3). Le dispositif de connexion comprend un écrou (23A), adapté pour recevoir une vis (21A) qui, par vissage, serre ensemble le membre conducteur électrique et la plage de raccordement, en les appuyant contre l'écrou, un noyau (25A), qui est thermiquement conducteur et qui est lié à l'écrou de manière à transférer la chaleur par conduction entre l'écrou et le noyau, et un support (27A) qui supporte l'écrou et le noyau de manière à la fois à maintenir l'écrou fixement en position et à transférer la chaleur par conduction entre le noyau et le support. Le support est réalisé dans un matériau qui émet un dégagement contenant des microparticules et/ou au moins un composé gazeux lorsque ce matériau est chauffé au-delà d'un seuil de température prédéterminé.

## Description

La présente invention concerne un dispositif de connexion électrique entre un membre conducteur électrique et une plage de raccordement d'un appareil électrique. Elle concerne également un appareil électrique comprenant un tel dispositif de connexion, ainsi qu'un tableau électrique comprenant un tel appareil électrique.

Dans le domaine des appareils électriques de puissance d'une installation électrique, les appareils électriques sont généralement montés sur un tableau électrique, lui-même logé dans un boitier ou une armoire électrique. Chaque appareil électrique comprend généralement une ou plusieurs plages de raccordement, auxquelles sont respectivement connectés des membres conducteurs électriques, tels que des câbles ou des tiges rigides - ou busbar en anglais -. Chaque membre conducteur électrique est, à une extrémité, connecté et fixé à l'une des plages de raccordement au moyen d'une vis serrée dans un écrou. Dans l'état de la technique, cet écrou est supporté et maintenu en position par un support en matière plastique. Ce support est rapporté fixement au carter de l'appareil électrique. A l'état monté, la vis traverse le membre conducteur électrique et la plage de raccordement et se visse dans l'écrou en appuyant le membre conducteur électrique contre la plage de raccordement pour assurer la connexion. Si la connexion entre le membre conducteur électrique et la plage de raccordement est mal serrée, la résistance électrique de la connexion augmente, et lorsque qu'un courant circule au travers de cette connexion, un échauffement localisé se produit au niveau de cette connexion, ce qui est une source potentielle de dysfonctionnement, voire d'accident si l'échauffement dépasse un certain seuil. Pour des raisons normatives et/ou de sécurité, il est donc nécessaire de surveiller la qualité des connexions électriques, notamment en surveillant l'échauffement de ces connexions.

Il est ainsi connu d'installer des capteurs de température au sein du tableau électrique pour une surveillance en continu des connexions électriques. Cependant ces capteurs, leur mise en place et l'exploitation de leurs mesures ont un coût élevé.

Par exemple, JP2002081998A décrit des dispositifs de détection utilisant une fibre optique installée proche des connexions électriques et une unité de commande transmettant de la lumière à la fibre optique. Une température anormale est détectée par la perte de transmission lumineuse de la fibre optique.

Il est aussi connu d'effectuer des prises de vues par caméra thermique au cours de visites d'inspection, afin de détecter des points chauds d'un tableau électrique. Seulement le tableau électrique doit être ouvert pour prendre les prises de vues, et par sécurité le tableau électrique ne fonctionne pas à pleine puissance, aussi les prises de vues ne reflètent pas fidèlement le fonctionnement normal des appareils électriques. D'autre part, l'ouverture du tableau électrique génère des déplacements d'air qui tendent à refroidir les appareils électriques montés sur ce tableau.

Il est également décrit dans US7712431 B2 des dispositifs de détection utilisant des éléments thermochromatiques changeant de couleur en fonction de leur température.

Une autre approche consiste à recourir à un dispositif de détection capable de détecter des gaz, des microparticules et des composés organiques volatiles, comme décrit dans EP3512056B1. En pratique, un dégagement gazeux ou particulaire, qui est anormalement émis par un équipement électrique, non précisé, est détecté par un capteur du dispositif de détection qui active alors une alarme.

Le but de la présente invention est de proposer un dispositif de connexion qui, tout en étant pratique d'utilisation, puisse efficacement signaler une mauvaise connexion.

A cet effet, l'invention a pour objet un dispositif de connexion électrique entre un membre conducteur électrique et une plage de raccordement d'un appareil électrique, le dispositif de connexion comprenant :
- un écrou, thermiquement conducteur et pourvu de première et seconde faces opposées l'une à l'autre suivant un axe, les première et seconde faces étant reliées l'une à l'autre par un taraudage de l'écrou, qui s'étend suivant l'axe et qui est adapté pour recevoir une vis qui, par vissage dans le taraudage, serre ensemble le membre conducteur électrique et la plage de raccordement, en les appuyant contre la première face de l'écrou,
- un noyau, qui est thermiquement conducteur et qui est lié à la seconde face de l'écrou de manière à transférer la chaleur par conduction entre l'écrou et le noyau, et
- un support, qui est adapté pour être rapporté fixement à un carter électriquement isolant de l'appareil électrique et qui supporte l'écrou et le noyau de manière à la fois à maintenir l'écrou fixement en position et à transférer la chaleur par conduction entre le noyau et le support, le support étant réalisé dans un matériau qui émet un dégagement contenant des microparticules et/ou au moins un composé gazeux lorsque ce matériau est chauffé au-delà d'un seuil de température prédéterminé

Grâce à l'invention, lorsqu'une connexion, entre un membre conducteur électrique et une plage de raccordement, est mal serrée, un échauffement se produit et chauffe le support qui émet alors un dégagement contenant des microparticules et/ou au moins un composé gazeux. Cet échauffement est généré par la résistance, induite par l'air dans l'espace entre le membre conducteur électrique et la plage de raccordement en raison du mauvais serrage, lorsqu'un courant circule à travers la connexion entre le membre conducteur électrique et la plage de raccordement. Au sein du dispositif de connexion conforme à l'invention, l'échauffement est transmis par conduction thermique de l'écrou au noyau, puis le noyau transmet par conduction thermique cet échauffement au support via une grande interface thermique que représente leur surface en contact, notamment lorsque le noyau est avantageusement enveloppé par le support, notamment par un matériau qui émet des gaz détectables en cas de surchauffe. Le dégagement généré par la surchauffe est ainsi détectable par un capteur de gaz et de particules situé en haut de tableau sans avoir besoin d'ouvrir le tableau électrique. Il est aussi intéressant de relever que le dégagement est immédiat lors d'un dysfonctionnement. En effet, la conduction thermique est un effet physique immédiat en présence de bons conducteurs thermiques, comme l'écrou et le noyau du dispositif de connexion. L'échauffement au niveau de la connexion est donc immédiatement transmis au support qui dégaze.

Suivant d'autres aspects avantageux de l'invention, le dispositif de connexion conforme à l'invention comprend une ou plusieurs des caractéristiques suivantes, prises isolément ou suivant toutes les combinaisons techniquement possibles :
- la première face de l'écrou est plane et s'étend perpendiculairement à l'axe.
- l'écrou et le noyau sont deux pièces distinctes, et le noyau est pourvu d'une face d'extrémité axiale, qui s'étend transversalement à l'axe et qui agencée en contact axialement contre la seconde face de l'écrou.
- la seconde face de l'écrou et la face d'extrémité axiale du noyau sont planes et s'étendent perpendiculairement à l'axe.
- le noyau est électriquement isolant, notamment en céramique.
- le noyau est pourvu d'une extrémité libre, qui est opposée suivant l'axe à la face d'extrémité axiale et qui émerge du support.
- le noyau est venu de matière avec l'écrou depuis la seconde face de l'écrou, et l'écrou et le noyau sont électriquement conducteurs, notamment en métal.
- le noyau est pourvue d'une face latérale, qui est globalement cylindrique en étant sensiblement centrée sur l'axe, et qui est au moins partiellement recouverte en contact par le support.

L'invention a également pour objet un appareil électrique, par exemple un dispositif de commutation, comprenant :
- un carter électriquement isolant,
- au moins une plage de raccordement, qui forme une entrée électrique ou une sortie électrique de l'appareil électrique, et
- pour la ou chaque plage de raccordement, un dispositif de connexion, tel que défini ci-avant, entre un membre conducteur électrique et la plage de raccordement.

L'invention a en outre pour objet un tableau électrique comprenant :
- un appareil électrique tel que défini ci-avant, et
- pour la ou chaque plage de raccordement, un membre conducteur électrique qui est connecté à la plage de raccordement par le dispositif de connexion.

L'invention a aussi pour objet un tableau électrique comprenant :
- un appareil électrique comprenant :
   - un carter électriquement isolant,
   - au moins une plage de raccordement, qui forme une entrée électrique ou une sortie électrique de l'appareil électrique, et
   - pour la ou chaque plage de raccordement, un membre conducteur électrique qui est connecté à la plage de raccordement par le dispositif de connexion, tel que défini ci-avant, pourvu d'une extrémité libre dépassant du support, et
- une plaque sur laquelle l'appareil électrique est fixé, la plaque étant thermiquement conductrice et étant en contact avec l'extrémité libre du noyau du dispositif de connexion de manière à transférer la chaleur par conduction thermique entre le noyau et la plaque.

L'invention apparaîtra plus clairement à la lecture de la description qui va suivre, donnée uniquement à titre d'exemple non limitatif, et faite en référence aux dessins dans lesquels :
[Fig. 1] la figure 1 est une vue schématique en perspective d'un tableau électrique conforme à l'invention ;
[Fig. 2] la figure 2 une coupe partielle de la figure 1, selon le plan Il de la figure 1 ;
[Fig. 3] la figure 3 est une vue en perspective d'une partie du tableau électrique de la figure 1 ;
[Fig. 4] la figure 4 est un éclaté des composants montrés à la figure 3 ; et
[Fig. 5] la figure 5 est une vue similaire à celle de la figure 2, illustrant un mode de réalisation alternatif.

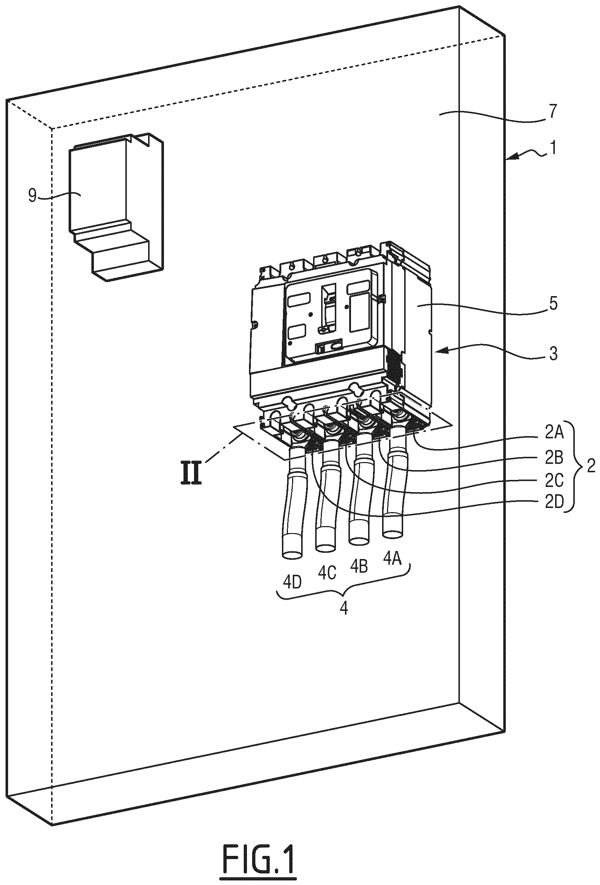

Un tableau électrique 1, selon un premier mode de réalisation, représenté sur la figure 1, comprend un appareil électrique 3, qui va être détaillé ci-après, et une plaque 7 sur laquelle l'appareil électrique 3 est fixé. La plaque 7 forme au moins en partie un fond arrière du tableau 1.

Pour des raisons qui apparaitront plus loin, la plaque 7 est avantageusement réalisée dans un matériau thermiquement conducteur, de préférence en métal.

L'appareil électrique 3 est, préférentiellement mais non limitativement, un disjoncteur, ici triphasé. Ce disjoncteur permet, de manière connue en soi, de faire transiter un courant électrique à une installation électrique et de couper le courant électrique en cas de court-circuit ou de surintensité dans l'installation électrique.

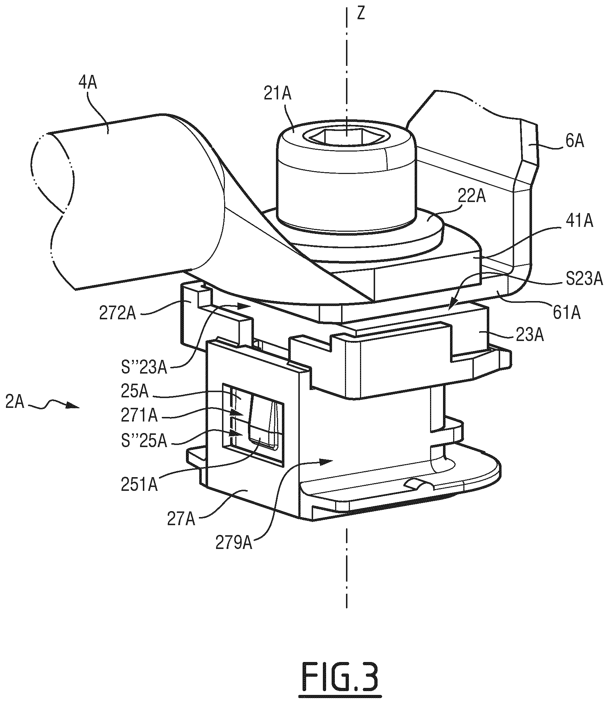

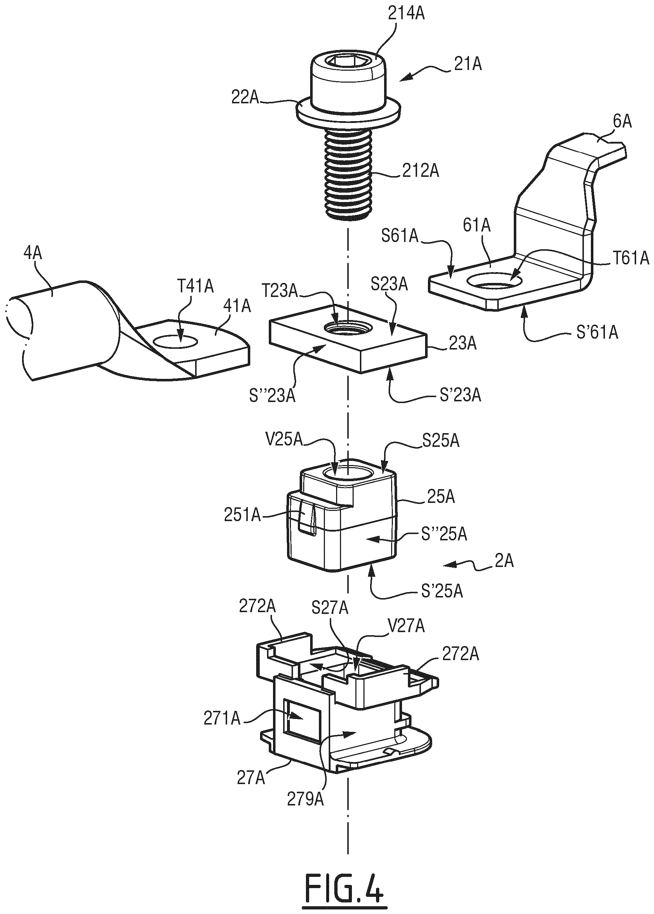

Afin d'alimenter l'appareil électrique 3 ou de faire transiter par ce dernier un courant électrique, ici triphasé, le tableau électrique 1 comprend un ou plusieurs membres conducteurs électriques 4 qui relient l'appareil électrique 3 à un réseau électrique, par exemple un réseau d'alimentation. Lorsque le réseau précité est triphasé, comme dans l'exemple considéré ici, les membres conducteurs électriques 4 incluent trois membres conducteurs électriques de phase, qui sont respectivement référencés 4A, 4B et 4C sur la figure 1, et un membre électrique conducteur de neutre, référencé 4D. En pratique, les membres conducteurs électriques 4A, 4B, 4C et 4D sont des câbles, comme illustré aux figures 1, 2, 3 et 4, ou bien des barres souples ou rigides, couramment appelés dans le domaine « busbar » selon la terminologie anglaise.

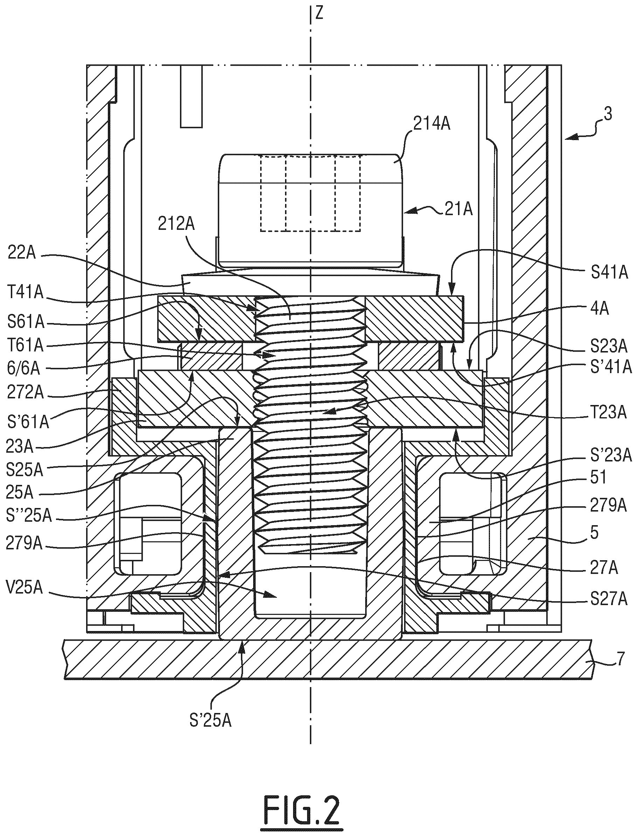

On décrit ci-après plus en détail le membre conducteur électrique 4A, étant entendu que cette description est applicable aux autres membres conducteurs électriques 4B, 4C et 4D. Comme visible sur les figures 2 à 4, le conducteur électrique 4A comprend, à son extrémité le reliant à l'appareil électrique 3, une cosse 41A. Cette cosse 41A comprend deux faces principales S41A et S'41A, qui sont parallèles entre elles et qui sont opposées l'une à l'autre suivant un axe géométrique, noté Z sur les figures 2 à 4 sur lesquelles cet axe s'étend à la verticale, étant entendu que cette orientation spatiale n'est pas limitative. La cosse 41A est traversée selon l'axe Z, de part en part, par un trou T41A.

Avantageusement, les deux faces principales S41A et S'41A de la cosse 41A sont planes et normales à l'axe Z.

En revenant à la description de l'appareil électrique 3, ce dernier comporte un carter 5 qui forme une enveloppe électriquement isolante à l'intérieur de laquelle sont agencés des composants de l'appareil électrique 3, assurant une fonction électrique principale de l'appareil électrique 3, telle qu'une fonction de transit électrique et de coupe-circuit dans le cas où l'appareil électrique 3 est un disjoncteur.

Le carter 5 est ainsi réalisé en un matériau électriquement isolant, typiquement une matière plastique, en particulier moulée.

L'appareil électrique 3 comporte également une ou plusieurs plages de raccordement 6, ici au nombre de quatre en égard au fait que l'appareil électrique 3 est triphasé. Les plages de raccordement 6 permettent la connexion de l'appareil électrique 3 avec les membres conducteurs 4, en formant ainsi une entrée électrique ou une sortie électrique pour l'appareil électrique 3, par laquelle le courant circule pour atteindre les composants précités qui, à l'intérieur du carter 5, assurent la fonction électrique principale de l'appareil électrique 3. A cet effet, les plages de raccordement 6 sont dans un matériau électriquement conducteur, préférentiellement du métal.

Les plages de raccordement 6 sont respectivement associées aux membres conducteurs électriques 4A à 4D de manière que chacune des plages de raccordement 6 et le membre conducteur électrique associé à la plage de raccordement 6 considérée sont connectés électriquement l'un à l'autre par un dispositif de connexion 2, détaillé par la suite. Sur la figure 1, on note 2A le dispositif de connexion entre le membre conducteur électrique 4A et la plage de raccordement 6 associée, cette dernière étant visible sur les figures 2 à 4 en y étant référencée 6A ; on note 2B le dispositif de connexion entre le membre conducteur électrique 4B et la plage de raccordement 6 associée ; on note 2C le dispositif de connexion entre le membre conducteur électrique 4C et la plage de raccordement 6 associée ; on note 2D le dispositif de connexion entre le membre conducteur électrique 4D et la plage de raccordement 6 associée. En pratique, les dispositifs de connexion 2A, 2B, 2C et 2D sont similaires, voire identiques entre eux, de sorte que, plus bas et en référence aux figures 2à 4, seul le dispositif de connexion 2A sera décrit plus en détail, cette description étant applicable aux autres dispositifs de connexion 2B, 2C et 2D.

On décrit plus en détail la plage de raccordement 6A, étant entendu que, en pratique cette description est applicable aux trois autres plages de raccordement. Comme bien visible sur les figures 2, 3 et 4, la plage de raccordement 6A comprend une extrémité 61A ayant deux faces principales S61A et S'61A, qui sont parallèles entre elles et qui sont opposées l'une à l'autre suivant un axe géométrique, qui, en service, c'est-à-dire lorsque la plage de raccordement 6A est connectée au membre conducteur 4A par le dispositif de connexion 2A, est confondu, à des jeux près, avec l'axe Z et qui sera donc considéré comme étant l'axe Z par la suite par commodité. L'extrémité 61A est traversée selon l'axe Z, de part en part, par un trou T61A.

Avantageusement, les deux faces principales S61A et S'61A de la plage de raccordement 6A sont planes et normales à l'axe Z.

Lorsque la face principale S61A de la plage de raccordement 6A est en contact avec la face principale S'41A du membre conducteur électrique 4A, la connexion électrique est assurée par contact, ici plan-plan, entre la plage de raccordement 6A et le membre conducteur électrique 4A.

Comme bien visible sur les figures 2, 3 et 4, le dispositif de connexion 2A comprend ici une vis 21A, une rondelle de serrage 22A, un écrou 23A, un noyau 25A et un support 27A.

La vis 21A permet de serrer le dispositif de connexion 2A. A cet effet, la vis 21A comporte une tige filetée 212A et une tête 214A, qui sont centrées sur un axe géométrique, qui, en service, est confondu, à des jeux près, avec l'axe Z et qui sera donc considéré comme étant l'axe Z par la suite par commodité. Avantageusement, la vis 21A est en métal.

La rondelle de serrage 22A permet de répartir les efforts de serrage appliqués par la vis 21A. La rondelle de serrage 22A est conçue pour, en service, être montée coaxialement autour de la tige filetée 212A. Avantageusement, la rondelle de serrage 22A est en métal.

L'écrou 23A permet l'assemblage vissé de la vis 21A au sein du dispositif de connexion 2A. L'écrou 23A comprend une première face S23A et une seconde face S'23A, qui sont parallèles entre elles et qui sont opposées l'une à l'autre suivant un axe géométrique, qui, en service, est confondu, à des jeux près, avec l'axe Z et qui sera donc considéré comme étant l'axe Z par la suite par commodité. Les première et seconde faces S23A et S'23A sont reliées l'une à l'autre par une face latérale S"23 de l'écrou. L'écrou 23A est traversé selon l'axe Z par un trou taraudé T23A reliant l'une à l'autre les faces S23A et S'23A.

L'écrou 23A est dans un matériau thermiquement conducteur, de préférence en métal.

Avantageusement, l'écrou 23A est de forme parallélépipédique et ses deux faces S23A et S'23A sont planes et normales à l'axe Z. Avantageusement, le trou taraudé T23A est centré sur les faces S23A et S'23A.

Lorsque la première face S23A de l'écrou 23A est en contact avec la face principale S'61A de la plage de raccordement 6A, la connexion thermique est assurée par contact, ici plan-plan, entre l'écrou 23A et la plage de raccordement 6A.

Le noyau 25A permet de transmettre la chaleur par conduction thermique au sein du dispositif de connexion 2A. Le noyau 25A, qui est distinct de l'écrou 23A, est pourvu de deux faces d'extrémité axiale S25A et S'25A, qui sont parallèles entre elles et qui sont opposées l'une à l'autre suivant un axe géométrique, qui, en service, est confondu, à des jeux près, avec l'axe Z et qui sera donc considéré comme étant l'axe Z par la suite par commodité.

Le noyau 25A est dans un matériau électriquement isolant et thermiquement conducteur, avantageusement en céramique.

Avantageusement, les faces d'extrémité axiale S25A et S'25A sont planes et normales à l'axe Z.

En service, la face d'extrémité axiale S25A du noyau 25A est conçue pour être en contact avec la seconde face S'23A de l'écrou 23A, en assurant une conduction thermique par contact, ici plan-plan, entre le noyau 25A et l'écrou 23A. Egalement en service, la face d'extrémité axiale S'25A du noyau 25A est conçue pour être en contact avec la plaque 7, en assurant une conduction thermique par contact, ici plan-plan, entre le noyau 25A et la plaque 7.

Par ailleurs, Les faces d'extrémité axiale S25A et S'25A sont reliées l'une à l'autre par une face latérale S"25A du noyau 25A. Avantageusement, cette face latérale S"25 présente une dimension suivant l'axe Z qui est bien supérieure à la dimension axiale correspondante de l'écrou, notamment supérieure au double, voire au triple de la dimension axiale correspondante de l'écrou. Egalement de manière avantageuse, la face latérale S"25 est globalement cylindrique, en étant sensiblement centrée sur l'axe Z et ayant ici une section transversale à profil sensiblement rectangulaire à coins arrondis.

De plus, le noyau 25A délimite avantageusement un volume interne V25A, qui débouche sur la face d'extrémité axiale S25A, en étant sensiblement centré sur l'axe Z, et qui est dimensionné pour recevoir partiellement la tige 212A de la vis 21A. Ainsi, à l'état assemblé du dispositif de connexion 2A, le volume interne V25 débouche dans le trou taraudé T23A de l'écrou 23A de manière que la vis 21A est partiellement reçue dans le volume interne V25A lors de son vissage dans le trou taraudé T23A.

Le support 27A permet de supporter l'écrou 23A et le noyau 25A de manière à la fois à maintenir l'écrou 23A fixement en position et à transférer la chaleur par conduction entre le noyau 25A et le support 27A.

A cet effet, le support 27A comporte un volume interne V27A qui est centré sur un axe géométrique, qui, en service, est confondu, à des jeux près, avec l'axe Z et qui sera donc considéré comme étant l'axe Z par la suite par commodité. Le volume interne 27A délimite une face interne S27A qui est ajustée de manière complémentaire à la face latérale S"25A du noyau 25, et ce avantageusement tout autour de l'axe Z. Ainsi, lorsque le noyau 25A est reçu dans le volume interne V27A du support 27A, la forme de la face interne S27A du support 27A coïncide avec la forme de la face latérale S"25A de sorte que le support 27A recouvre majoritairement, voire quasi-totalement la face latérale S"25 pour qu'il y ait un maximum de contact entre la paroi interne S27A et la face latérale S"25A, en particulier sans lame d'air entre eux.

Ici, la section transversale sensiblement rectangulaire de la face latérale S"25A fait que, par complémentarité de formes avec la face interne 27A du support 27A, le noyau 25A et le support 27 A sont, à l'état assemblé, bloqué en rotation autour de l'axe Z l'un par rapport à l'autre. Pour retenir au moins temporairement le noyau 25A et le support 27 l'un par rapport à l'autre suivant l'axe Z, le noyau 23A et le support 27 sont ici pourvus de moyens de retenue ad hoc, incluant au moins une protubérance extérieure 251A du noyau 25A et un ajour 271A du support 27A, qui coopèrent l'un avec l'autre par complémentarité de formes. Bien entendu, d'autres formes de réalisation sont envisageables pour les moyens de retenue précités.

A l'une de ses extrémités axiales, qui, en service, est tournée vers l'écrou 23A, le support 27A est pourvu de saillies 272A qui sont conçues, notamment de par leur forme et dimension, pour bloquer l'écrou 23A en rotation autour de l'axe Z à l'état assemblé du dispositif de connexion 2A. En particulier, lorsque que l'écrou 23A est positionné sur le support 27A, les saillies 272A coïncident avec la forme de la face latérale S"23A de l'écrou 25A et bloquent, par contact avec la face latérale S"23 de l'écrou 23A, la rotation, autour de l'axe Z, de l'écrou 23A. Les spécificités de ces saillies 272A ne sont pas limitatives, étant entendu qu'elles tirent avantageusement profit de la forme parallélépipédique de l'écrou 23A.

Plus généralement, le support 27A est pourvu d'aménagements qui permettent à la fois de retenir en position par rapport à lui l'écrou 23A et le noyau 25A et de former une interface de contact substantielle, typiquement cylindrique centrée sur l'axe Z, entre lui et le noyau 25A aux fins de conduction thermique entre eux. En pratique, de multiples formes de réalisation sont envisageables pour ces aménagements, autres que le volume interne 27A, les moyens de retenue précités et les saillies 272A, ayant été détaillés ci-dessus à titre d'exemple. Ainsi, suivant une variante avantageuse, non illustrée aux figures, le support 27A est surmoulé sur le noyau 25A et/ou sur l'écrou 23A.

Dans tous les cas, dans la forme de réalisation illustrée aux figures 1 à 4, le noyau 25A et le support 27A sont conçus pour, à l'état assemblé du dispositif de connexion 2A, laisser la face d'extrémité S'25A du noyau être disposer à l'extérieur du noyau 27A, comme bien visible sur la figure 2. La face S'25A du noyau 25 forme ainsi une extrémité libre de ce dernier, qui émerge du support 27A.

Par ailleurs, le support 27A assure une autre fonction au sein du dispositif de connexion 2A, à savoir permettre d'être rapporté fixement au carter 5 de l'appareil électrique 3 et ainsi permettre d'assembler le dispositif de connexion 2 à ce carter 5.

A cet effet, le support 27A comporte ici des gorges externes 279A adaptées pour recevoir, avantageusement par coulissement suivant leur direction longitudinale, des rails complémentaires 51 du carter 5, notamment prévus pour évacuer des gaz depuis une chambre de coupure électrique se trouvant à l'intérieur du carter 5 dans le cas où l'appareil électrique 3 est un disjoncteur.

Bien entendu, de multiples autres formes de réalisation sont envisageables pour les aménagements externes du support 27A, qui permettent de rapporter fixement ce dernier au carter 5.

Dans tous les cas, le support 27A est réalisé dans un matériau émettant un dégagement contenant des microparticules et/ou au moins un composé gazeux lorsqu'il est chauffé au-delà d'un seuil de température prédéterminé. A titre d'exemples, ce matériau est un polymère thermoplastique, par exemple du Polychlorure de vinyle, dit aussi PVC, ou bien du polyamide 6/6, du polyéthylène téréphtalate, noté PET, du polybutylène téréphtalate, noté PBT.

Avantageusement, ce matériau du support 27A est électriquement isolant.

Afin de connecter le membre conducteur électrique 4A et la plage de raccordement 6A, la vis 2A est agencée selon l'axe Z au travers de successivement la rondelle 22A, le membre conducteur électrique 4A et la plage de raccordement 6A et est serrée dans l'écrou 23A, en se logeant partiellement dans le volume interne V25A du noyau 25A. Ainsi, le membre conducteur électrique 4A et la plage de raccordement 6A sont pressés entre la rondelle 22A et l'écrou 23A. Le contact entre le membre conducteur électrique 4A et la plage de raccordement 6A assure leur bonne connexion électrique. La nature d'isolant électrique des matériaux respectifs du carter 5 et du noyau 2A permet d'isoler électriquement la connexion de l'extérieur et d'éviter les risques électriques pour un utilisateur.

En cas de mauvais serrage, le contact entre le membre conducteur électrique 4A et la plage de raccordement 6A n'est que partiellement assuré et l'air dans l'espace entre le membre conducteur électrique 4A et la plage de raccordement 6A crée une résistance électrique produisant un échauffement lorsqu'elle est traversée par un courant électrique. L'échauffement est transmis de la plage de raccordement 6A à l'écrou 23A par conduction au travers de l'interface de contact entre la première face S23A de l'écrou 23A et la face principale S'61A de la plage de raccordement 6A. Le phénomène thermique est facilité par la nature de conducteur thermique des matériaux de la plage de raccordement 6A et de l'écrou 23A et par le contact plan-plan entre la plage de raccordement 6A et l'écrou 23A.

L'échauffement est ensuite transmis de l'écrou 23A au noyau 25A par conduction au travers de l'interface de contact entre la face d'extrémité axiale S25A du noyau 25A et la seconde face S'23A de l'écrou 23A. Le phénomène thermique est facilité par la nature de conducteur thermique des matériaux de l'écrou 23A et du noyau 25A et par le contact plan-plan entre l'écrou 23A et le noyau 25A.

L'échauffement est ensuite transmis du noyau 25A au support 27A par conduction au travers de l'interface de contact entre la face interne S27A du support 27A et la face latérale S"25 du noyau. Le phénomène thermique est facilité par la nature de conducteur thermique du matériau du noyau 25A et par la grande interface de contact qu'offre l'enveloppement de la face latérale S"25A du noyau 25A par la face interne S27A du support 27A.

Le support 27A ainsi chauffé émet, dès lors qu'il est porté à une température supérieure au seuil de température prédéterminé précité, un dégagement contenant des microparticules et/ou au moins un composé gazeux. Lorsque l'appareil fonctionne à pleine puissance, l'émission du dégagement est immédiate lors d'un mauvais serrage grâce à la rapidité de l'effet physique de conduction thermique. Un détecteur 9 de gaz, de microparticules et/ou de composés organiques volatiles, comme décrit dans EP3512056B1, est avantageusement inclut dans le tableau électrique 1 et son alarme se déclenche lors de l'émission du dégagement.

Le contact plan-plan entre la plaque 7 et la face S'25A du noyau 25A permet avantageusement de refroidir, en dissipant la chaleur par conduction thermique, l'appareil électrique 3 et d'éviter que l'échauffement soit trop conséquent et dégrade l'appareil électrique 3.

En variante non représentée, la position du membre conducteur électrique 4 et de la plage de raccordement est inversée, c'est-à-dire que la face principale S41A du membre conducteur 4A est en contact avec la face principale S'61A de la plage de raccordement 6A et que la première face S23A de l'écrou 23A est en contact avec la face principale S'41A du membre conducteur électrique S"41. Le fonctionnement de l'appareil électrique 3 est identique, cette variante permet à l'utilisateur de positionner le membre conducteur électrique 4A et la plage de raccordement 6A dans l'ordre le plus pratique pour réaliser la connexion.

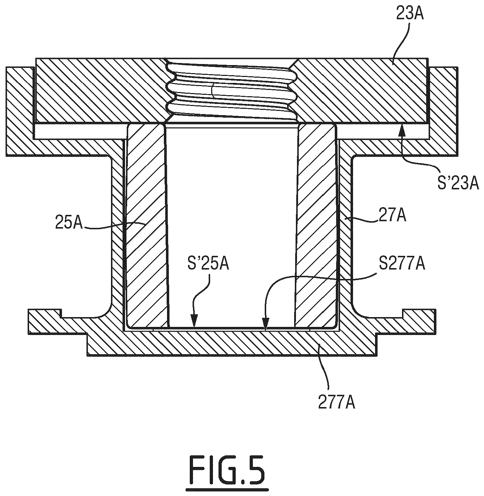

La figure 5 illustre un deuxième mode de réalisation du dispositif de connexion 2A, identique au premier mode de réalisation sauf pour les caractéristiques ci-après. Le support 27A comporte une paroi de fond 277A qui s'étend transversalement à l'axe Z en fermant le volume interne V27A. Le noyau 25A n'émerge pas du support 27A et sa face S'25A est recouverte par une face interne S277A de la paroi de fond 277A. En service, la paroi de fond se retrouve interposée, suivant l'axe Z, entre le noyau 25A et la plaque 7.

Il est dès lors possible de prévoir, dans ce deuxième mode de réalisation, que le noyau 25A et l'écrou 23 soient électriquement conducteurs, notamment en métal, sans risque pour l'utilisateur. En variante à ce deuxième mode de réalisation, non représentée, le noyau 25A est alors avantageusement venu de matière avec l'écrou 23A depuis la seconde face S'23A de l'écrou 23A. Autrement dit, l'écrou 23A et le noyau 25A sont alors monobloc.

Par ailleurs, on notera que la vis 21A et la rondelle de serrage 22A peuvent être des pièces préexistantes, appartenant à l'appareil électrique 3 au niveau d'un dispositif de connexion de l'art antérieur, qu'on peut d'ailleurs remplacer par le dispositif de connexion 2.

Toute caractéristique décrite ci-avant pour un mode de réalisation ou une variante est applicable aux autres modes de réalisation et variantes décrits ci-avant, pour autant que cela est techniquement possible.

## Revendications

1. Dispositif de connexion (2) électrique entre un membre conducteur électrique (4) et une plage de raccordement (6) d'un appareil électrique, le dispositif de connexion (2) comprenant :
- un écrou (23A), thermiquement conducteur et pourvu de première et seconde faces (S23A, S'23A) opposées l'une à l'autre suivant un axe (Z), les première et seconde faces (S23A, S'23A) étant reliées l'une à l'autre par un taraudage (T23A) de l'écrou (23A), qui s'étend suivant l'axe (Z) et qui est adapté pour recevoir une vis (21A) qui, par vissage dans le taraudage, serre ensemble le membre conducteur électrique (4) et la plage de raccordement (6), en les appuyant contre la première face (S23A) de l'écrou (23A),
- un noyau (25A), qui est thermiquement conducteur et qui est lié à la seconde face de l'écrou (S23A) de manière à transférer la chaleur par conduction entre l'écrou (23A) et le noyau (25A), et
- un support (27A), qui est adapté pour être rapporté fixement à un carter (5) électriquement isolant de l'appareil électrique (3) et qui supporte l'écrou (23A) et le noyau (25A) de manière à la fois à maintenir l'écrou (23A) fixement en position et à transférer la chaleur par conduction entre le noyau (25A) et le support (27A), le support étant réalisé dans un matériau qui émet un dégagement contenant des microparticules et/ou au moins un composé gazeux lorsque ce matériau est chauffé au-delà d'un seuil de température prédéterminé.

2. Dispositif de connexion (2) selon la revendication 1, dans lequel la première face de l'écrou (S23A) est plane et s'étend perpendiculairement à l'axe (Z).

3. Dispositif de connexion (2) selon l'une quelconque des revendications 1 et 2, dans lequel l'écrou (23A) et le noyau (25A) sont deux pièces distinctes, et dans lequel le noyau est pourvu d'une face d'extrémité axiale (S25A), qui s'étend transversalement à l'axe (Z) et qui agencée en contact axialement contre la seconde face de l'écrou (S'23A).

4. Dispositif de connexion (2) suivant la revendication 3, dans lequel la seconde face de l'écrou (S'23A) et la face d'extrémité axiale (S25A) du noyau sont planes et s'étendent perpendiculairement à l'axe (Z).

5. Dispositif de connexion (2) selon l'une quelconque des revendications 3 à 4, dans lequel le noyau (25A) est électriquement isolant, notamment en céramique.

6. Dispositif de connexion (2) selon la revendication 5, dans lequel le noyau (25A) est pourvu d'une extrémité libre (S'25A), qui est opposée suivant l'axe à la face d'extrémité axiale (S25A) et qui émerge du support (27A).

7. Dispositif de connexion (2) selon l'une quelconque des revendications 1 et 2, dans lequel le noyau (25A) est venu de matière avec l'écrou (23A) depuis la seconde face de l'écrou (S'23A), et dans lequel l'écrou (23A) et le noyau (25A) sont électriquement conducteurs, notamment en métal.

8. Dispositif de connexion (2) selon l'une quelconque des revendications 1 à 7, dans lequel le noyau (25A) est pourvue d'une face latérale (S"25A), qui est globalement cylindrique en étant sensiblement centrée sur l'axe (Z), et qui est au moins partiellement recouverte en contact par le support (27A).

9. Appareil électrique (3), par exemple un dispositif de commutation, comprenant :
- un carter (5) électriquement isolant,
- au moins une plage de raccordement (6), qui forme une entrée électrique ou une sortie électrique de l'appareil électrique, et
- pour la ou chaque plage de raccordement (6), un dispositif de connexion (2) entre un membre conducteur électrique (4) et la plage de raccordement (6), le dispositif étant selon l'une quelconque des revendications 1 à 8.

10. Tableau électrique (1) comprenant :
- un appareil électrique (3) selon la revendication 9, et
- pour la ou chaque plage de raccordement (6), un membre conducteur électrique (4) qui est connecté à la plage de raccordement par le dispositif de connexion (2).

11. Tableau électrique (1) comprenant :
- un appareil électrique (3) comprenant :
- un carter (5) électriquement isolant,
- au moins une plage de raccordement (6), qui forme une entrée électrique ou une sortie électrique de l'appareil électrique, et
- pour la ou chaque plage de raccordement (6), un membre conducteur électrique (4) qui est connecté à la plage de raccordement par le dispositif de connexion (2) selon la revendication 6, et
- une plaque (7) sur laquelle l'appareil électrique (3) est fixé, la plaque étant thermiquement conductrice et étant en contact avec l'extrémité libre (S'25A) du noyau (25A) du dispositif de connexion (2) de manière à transférer la chaleur par conduction thermique entre le noyau (25A) et la plaque (7).
